# EUROPEAN PATENT APPLICATION

(11) **EP 3 744 347 A1**
(43) Date of publication of application: **02.12.2020**
(21) Application number: 19744152.0
(22) Date of filing: 23.01.2019
(51) Int. Cl.: A61K 45/00, A61K 9/127, A61K 9/16, A61K 31/366, A61K 31/565, A61K 35/545, A61K 47/34, A61K 47/46, A61P 5/30, A61P 17/02, A61P 17/14, A61P 43/00, C07K 14/575, C12N 5/0775

(54) **COMPOSITION FOR SKIN DISEASES TREATMENT USE**

(30) Priority: 23.01.2018 JP 2018008703
(71) Applicant: Novumcella Inc., Tokyo 105-0004 (JP)
(72) Inventor: II Masaaki, Ibaraki-shi, Osaka 567-0868 (JP)
(74) Representative: Steinecke, Peter
(86) International application number: PCT/JP2019/001969
(87) International publication number: WO 2019/146612

(57) **Abstract**

The present invention provides an excellent composition for therapeutically treating a skin disease (e.g., a drug, a quasi-drug or a cosmetic). The composition for therapeutically treating a skin disease according to the present invention contains exosomes secreted from stem cells (for example, adipose-derived stem cells), in which the stem cells contain drug-encapsulating nanoparticles comprising a drug (for example, statin, estrogen, retinoic acid and ascorbic acid) encapsulated in nanoparticles containing a bioabsorbable polymer.

SEQUENCE LISTING

<110>  Novumcella Inc.

<120>  Composition for treating skin disease

<130>  PN57-9001WO

<150>  JP 2018-8703
<151>  2018-01-23

<160>  8

<170>  PatentIn version 3.5

<210>  1
<211>  18
<212>  DNA
<213>  Artificial Sequence

<220>
<223>  VEGF-A forward primer

<400>  1
ttactctcac ctgcttct                                                    18


<210>  2
<211>  18
<212>  DNA
<213>  Artificial Sequence

<220>
<223>  VEGF-A reverse primer

<400>  2
ctgcttcttc caacaatg                                                    18


<210>  3
<211>  19
<212>  DNA
<213>  Artificial Sequence

<220>
<223>  VEGF-C forward primer

<400>  3
tcaaggacag aagagacta                                                   19


<210>  4
<211>  19
<212>  DNA
<213>  Artificial Sequence

<220>
<223>  VEGF-C reverse primer

<400>  4
ccacatctat acacacctc                                                   19

<210>  5
<211>  19
<212>  DNA
<213>  Artificial Sequence

<220>
<223>  FGF-2 forward primer

<400>  5
ttcttccaat gtctgctaa                                                    19


<210>  6
<211>  20
<212>  DNA
<213>  Artificial Sequence

<220>
<223>  FGF-2 reverse primer

<400>  6
gaccaattat ccaaactgag                                                   20


<210>  7
<211>  22
<212>  DNA
<213>  Artificial Sequence

<220>
<223>  HGF forward primer

<400>  7
cctctatgaa aacaaagact ac                                                22


<210>  8
<211>  18
<212>  DNA
<213>  Artificial Sequence

<220>
<223>  HGF reverse primer

<400>  8
ctgtgttcgt gtggtatc                                                     18

## Description

### Technical Field

The present invention relates to a composition for therapeutically treating a skin disease. More specifically, the present invention relates to a composition for therapeutically treating a skin disease, comprising adipose-derived stem cells containing drug-encapsulating nanoparticles or exosomes secreted from the adipose-derived stem cells.

### Background Art

Recently, studies for therapeutically treating various diseases by using pluripotent stem cells have been conducted. As the stem cells, e.g., embryonic stem cells (ES cells), mesenchymal somatic stem cells such as bone marrow-derived stem cells and adipose-derived stem cells, and induced pluripotent stem cells (iPS cells) are generally known and used in a wide variety of studies. For example, somatic stem cells are used in autologous cell transplantation, which has been aggressively studied in basic research and clinical trial for use in therapeutic treatment for many diseases such as inflammatory diseases, ischemic diseases, allergic diseases and neurodegenerative diseases, not only in western countries but also in Japan. Of the somatic stem cells, easy-to-handle adipose-derived stem cells, the study on which has rapidly progressed and clinical trials thereof in regenerative medical techniques for therapeutically treating various diseases has been widely conducted. In addition to use in regenerative medical techniques, adipose-derived stem cells are reported to have an enterocolitis suppression effect in a drug-induced enteritis mouse model (see, for example, Non Patent Literature 1).

As a means for improving therapeutic effect of the aforementioned adipose-derived stem cells on diseases, for example, Patent Literature 1 discloses a method of enhancing cellular function such as proliferative potential and cytokine synthetic potential by allowing adipose-derived stem cells to uptake statin-encapsulating nanoparticles.

On the other hand, other than use of adipose-derived stem cells themselves for disease treatment, use of culture supernatant of adipose-derived stem cells and exosomes released from adipose-derived stem cells as agents for therapeutically treating diseases, are also studied.

For example, Patent Literature 2 discloses cosmetics and therapeutic agents for skin diseases (e.g., therapeutic compositions against allergy, composition for hair growth or hair restoration) containing the culture supernatant of horse mesenchymal stem cells (e.g., adipose-derived stem cells).

The exosomes refer to membrane vesicles secreted from cells and responsible for transporting intracellular proteins and genetic information (e.g., mRNA and microRNA) outside the cells; thereby playing a role in local and systemic information transfer between cells. Exosomes have a wide variety of functions; for example, mediation of appropriate immune response against infectious pathogens and tumors, tissue repair, nerve transmission and transport of pathogenic proteins have been reported as the functions. It is also suggested that the function of exosomes differs depending on the cells from which the exosomes are derived. For example, exosomes derived from mesenchymal stem cells such as adipose-derived stem cells are reported to have a function to mitigate myocardial ischemia/reperfusion damage and a function to mitigate pulmonary hypertension in a hypoxic condition; whereas, exosomes derived from myocardial progenitor cells are reported to have a function to suppress apoptosis of myocardium (see, e.g., Non Patent Literatures 2 to 5). Because of this, exosomes secreted from different types of cells conceivably bring vary physiological functions and lead to development of therapeutic treatment for various diseases.

### Citation List

### Patent Literature

Patent Literature 1: JP 6110578 B (WO2016/076227)
Patent Literature 2: JP 6152205 B (JP 2018-24595 A)

### Non Patent Literatures

Non Patent Literature 1: Gonzalez, MA et al. Gastroenterology 136(2009), 978-989.
Non Patent Literature 2: Ruenn Chai Lai et al. Stem Cell Research (2010) 4, 214-222.
Non Patent Literature 3: Fatih arslan et al. Stem Cell Research (2013) 10, 301-312.
Non Patent Literature 4: Changjin Lee et al. Circulation. 2012;126: 2601-2611.
Non Patent Literature 5: Lucio Barile et al. Cardiovascular Research (2014) 103, 530-541.

### Summary of Invention

### Technical Problem

Although disclosure of the aforementioned prior-art documents, compositions showing sufficient efficacy in therapeutically treating skin diseases have not yet been obtained at present. In view of convenience, a composition for therapeutically treating a skin disease showing high efficacy even for external use, is desired.

An object of the present invention is to provide a composition for therapeutically treating a skin disease, having an excellent therapeutic effect.

### Solution to Problem

In order to attain the aforementioned object, the present inventors intensively conducted studies. As a result, they have found that when drug-encapsulating nanoparticles in which a drug is encapsulated in nanoparticles are incorporated in stem cells, exosomes secreted from the stem cells contain large amounts of components useful for therapeutic treatment for various skin diseases, i.e., the drug encapsulated in the nanoparticles and various factors produced in the stem cells, such as an angiogenic factor; and that if such exosomes are used as an active ingredient, a composition for therapeutically treating a skin disease, having excellent therapeutic effect on the skin disease and successfully used as an external preparation, can be prepared. Based on the findings, the present invention was accomplished.

More specifically, according to an aspect of the present invention, the following items are provided.
[Item 1] A composition for therapeutically treating a skin disease, comprising exosomes secreted from stem cells, wherein
the stem cells are stem cells containing drug-encapsulating nanoparticles in which a drug is encapsulated in nanoparticles comprising a bioabsorbable polymer.
[Item 2] The composition according to Item 1, wherein the stem cells are adipose-derived stem cells.
[Item 3] The composition according to Item 1 or 2, wherein the drug is effective for therapeutically treating a skin disease and enhances an expression level of a factor, which is produced by the stem cells and effective for therapeutically treating the skin disease.
[Item 4] The composition according to any one of Items 1 to 3, wherein the drug is statin.
[Item 5] The composition according to any one of Items 1 to 3, wherein the drug is estrogen.
[Item 6] The composition according to any one of Items 1 to 3, wherein the drug is vitamin A or a derivative thereof.
[Item 7] The composition according to any one of Items 1 to 3, wherein the drug is vitamin C or a derivative thereof.
[Item 8] The composition according to any one of Items 1 to 4, 6 and 7, wherein the skin disease is alopecia or atrichia.
[Item 9] The composition according to any one of Items 1 to 3, 5 and 6, wherein the skin disease is skin damage or skin ulcer.
[Item 10] The composition according to any one of Items 1 to 9, having dosage form for transdermal administration.
[Item 11] The composition according to any one of Items 1 to 10, wherein the composition is a drug, a quasi-drug or a cosmetic.

Note that, properties of the "exosomes secreted" from the "stem cells containing drug-encapsulating nanoparticles", particularly, the composition (components and amounts of components) of the content of the exosomes cannot be generally determined because the composition may vary depending on, e.g., the type of drug added in nanoparticles, the amount of drug-encapsulating nanoparticles (the concentration of drug-encapsulating nanoparticles in the culture medium for stem cells) in stem cells, and the time from addition of drug-encapsulating nanoparticles in stem cells up to collection of exosomes (passage number), as described later in the specification. Generally, the "exosomes secreted" from the "stem cells containing drug-encapsulating nanoparticles" contain the drug encapsulated in the drug-encapsulating nanoparticles in an effective amount for therapeutically treating a skin disease, and factors (e.g., a protein and a nucleic acid) expressed by the stem cells secreting the exosomes. Preferably, the amounts of factors (e.g., a protein and a nucleic acid) present in exosomes secreted from stem cells containing drug-encapsulating nanoparticles are large compared to the amounts of factors in exosomes secreted from stem cells (control) not containing drug-encapsulating nanoparticles. In other words, the amount of exosomes secreted from stem cells containing drug-encapsulating nanoparticles for use in producing the composition for therapeutically treating a skin disease of the present invention may be less than the amount of exosomes secreted from stem cells not containing drug-encapsulating nanoparticles in order to attain the same therapeutic effect.

According to another aspect of the present invention, there is provided a "method for therapeutically treating a skin disease, including administering (e.g., contacting with the skin or applying) an effective amount of exosomes secreted from stem cells containing drug-encapsulating nanoparticles in which a drug is encapsulated in nanoparticles comprising a bioabsorbable polymer". The invention concerning "method for therapeutically treating a skin disease" can be easily led by those skilled in the art from the invention concerning the "composition for therapeutically treating a skin disease" set forth, e.g., in Item 1. The matters in connection with the "composition for therapeutically treating a skin disease" specifically described in the specification can be similarly applied to the "method for therapeutically treating a skin disease".

According to another aspect of the present invention, there is provided "use of exosomes secreted from stem cells containing drug-encapsulating nanoparticles in which a drug is encapsulated in nanoparticles comprising a bioabsorbable polymer in preparation of a composition for therapeutically treating a skin disease". The invention concerning the "use" can be easily led by those skilled in the art from the invention concerning the "composition for therapeutically treating a skin disease" set forth, e.g., in Item 1. The matters in connection with the "composition for therapeutically treating a skin disease" specifically described in the specification can be similarly applied to the "method for therapeutically treating a skin disease"

According to another aspect of the present invention, there is provide a "method for producing a composition for therapeutically treating a skin disease, comprising the steps of: incorporating, in the stem cells, drug-encapsulating nanoparticles in which a drug is encapsulated in nanoparticles comprising a bioabsorbable polymer; and collecting exosomes secreted from the stem cells". The invention concerning the "method for producing a composition for therapeutically treating a skin disease" can be easily led by those skilled in the art based on the invention concerning the "composition for therapeutically treating a skin disease" set forth in, e.g., Item 1 and in view of matters in connected with the "composition for therapeutically treating a skin disease" specifically described in the specification. An embodiment where a first practitioner carries out the "step of incorporating, in the stem cells, drug-encapsulating nanoparticles in which a drug is encapsulated in nanoparticles comprising a bioabsorbable polymer" and a second practitioner receives the stem cells from the first practitioner and carries out the "step of collecting exosomes secreted from the stem cells", is also included in the "method for producing a composition for therapeutically treating a skin disease".

### Advantageous Effects of Invention

The composition for therapeutically treating a skin disease according to the present invention exerts a remarkably excellent therapeutic effect compared to compositions ordinarily used and are extremely useful in therapeutically treating various skin diseases such as alopecia or atrichia and skin damage or skin ulcer. Since the composition for therapeutically treating a skin disease according to the present invention contains exosomes, which have high transdermal absorbability, as an active ingredient, the composition can be provided as a drug, a quasi-drug or a cosmetic that can be used as an external preparation (to be transdermally administered).

### Brief Description of Drawings

Figure 1 shows photographs showing observation results of human adipose-derived stem cells by a confocal laser fluorescence microscope, after the stem cells were cultured in a medium having rhodamine red fluorescent dye-encapsulating PLA nanoparticles added therein at a final concentration of 20 µg/mL, 50 µg/mL, 80 µg/mL or 100 µg/mL, respectively for one hour or two hours.
Figure 2 shows photographs showing the uptake amount of rhodamine red fluorescent dye-encapsulating PLGA nanoparticles in mouse adipose-derived stem cells, measured by FACS, after the stem cells were cultured in a medium having rhodamine red fluorescent dye-encapsulating PLGA nanoparticles added therein at a final concentration of 20 µg/mL, 50 µg/mL, 75 µg/mL or 100 µg/mL, for 30 minutes.
Figure 3 shows a graph indicating measurement results, i.e., the amount of simvastatin released from human adipose-derived stem cells into a medium after the human adipose-derived stem cells were treated with 100 µg/mL simvastatin-encapsulating PLA nanoparticles for one hour.
Figure 4 shows a graph indicating measurement results, i.e., the amount of simvastatin released from mouse adipose-derived stem cells into a medium after the mouse adipose-derived stem cells were treated with 50 µg/mL simvastatin-encapsulating PLA nanoparticles for 30 minutes.
Figure 5 shows graphs indicating measurement results, i.e., angiogenic factor mRNA expression in adipose-derived stem cells treated with statin-encapsulating nanoparticles by quantitative PCR method, in which (a) shows RNA expression of angiogenic factor VEGF-A, (b) shows RNA expression of angiogenic factor VEGF-C, and (c) shows RNA expression of angiogenic factor FGF-2.
Figure 6 shows photographs showing hair growth and hair restoration effects of exosomes secreted from adipose-derived stem cells treated with statin-encapsulating nanoparticles.
Figure 7 shows photographs showing hair growth and hair restoration effects of exosomes secreted from adipose-derived stem cells treated with statin-encapsulating nanoparticles with hairless mice.
Figure 8 shows optical micrographs showing skin regeneration effect of exosomes secreted from adipose-derived stem cells treated with statin-encapsulating nanoparticles or estradiol-encapsulating nanoparticles.
Figure 9 shows a graph indicating VEGF(-A) gene expression in adipose-derived stem cells of a woman of 71 years old treated with estradiol-encapsulating PLGA nanoparticles.
Figure 10 shows a graph indicating HGF gene expression in adipose-derived stem cells of a woman of 71 years old treated with estradiol-encapsulating PLGA nanoparticles.
Figure 11 shows a graph indicating FGF2 gene expression in adipose-derived stem cells of a woman of 71 years old treated with estradiol-encapsulating PLGA nanoparticles.
Figure 12 shows a graph indicating VEGF(-A) gene expression in adipose-derived stem cells of a woman of 71 years old treated with vitamin C-encapsulating PLGA nanoparticles.
Figure 13 shows a graph indicating HGF gene expression in adipose-derived stem cells of a woman of 71 years old treated with vitamin C-encapsulating PLGA nanoparticles.
[Figure 14] The figure shows a graph indicating FGF2 gene expression in adipose-derived stem cells of a woman of 71 years old treated with vitamin C-encapsulating PLGA nanoparticles.
[Figure 15] The figure shows a graph indicating VEGF(-A) gene expression in adipose-derived stem cells of a woman of 71 years old treated with retinoic acid-encapsulating PLGA nanoparticles.
[Figure 16] The figure shows a graph indicating HGF gene expression in adipose-derived stem cells of a woman of 71 years old treated with retinoic acid-encapsulating PLGA nanoparticles.
[Figure 17] The figure shows a graph indicating FGF2 gene expression in adipose-derived stem cells of a woman of 71 years old treated with retinoic acid-encapsulating PLGA nanoparticles.

### Description of Embodiments

Now, embodiments for carrying out the present invention will be more specifically described below. In the following detailed description of the invention, preferable embodiments and embodiments disclosed by the drawings and Examples will be described as needed; however, the embodiments are basically just examples, which should not be construed as limiting the technical scope of the present invention.

The composition for therapeutically treating a skin disease according to the present invention contains exosomes secreted from stem cells containing drug-encapsulating nanoparticles in which a drug is encapsulated in nanoparticles comprising a bioabsorbable polymer (sometimes referred to as "nanoparticle-containing stem cells" in the specification).

### <Drug-encapsulating nanoparticles>

In the present invention, the "drug" to be encapsulated in nanoparticles, which are to be incorporated in stem cells for collecting exosomes, is a substance, which can be selected from various substances having a function effect on a human body, more specifically, substances that can act on components contained in exosomes to be secreted from stem cells and can produce a function effect of the present invention applicable to therapeutic treatment for a skin disease when the exosomes are used. The "components contained in exosomes secreted from stem cells" include the "drug" encapsulated in exosomes and various factors produced by the stem cells (in particular, factors such as an angiogenic factor, effective for therapeutically treating a skin disease). The "drug" itself has an "effect" on the components within exosomes in the sense of being integrated into the components within exosomes and preferably further enhancing the expression level of a factor produced by stem cells. The drug can be selected from compounds belonging to a peptide (including a peptide hormone) or a derivative thereof, a protein, a nucleic acid, a polysaccharide, a vaccine, an adjuvant and a water-soluble or lipid-soluble low-molecular weight compound. Alternatively, a drug known as a component in drugs, quasi-drugs or cosmetics and, in particular, a drug known as component in drugs, quasi-drugs or cosmetics effective for therapeutically treating skin diseases, may be used.

In a preferable embodiment of the present invention, the drug (low-molecular weight compound) to be encapsulated in nanoparticles is "statin". The "statin" in the present invention is a general term referring to all compounds serving as a HMG-CoA (3-hydroxy-3-methylglutaryl-coenzyme A) reductase inhibitor. Examples of the statin that can be used in the present invention include simvastatin, rosuvastatin, pitavastatin, atorvastatin, cerivastatin, fluvastatin, pravastatin, lovastatin and mevastatin. Many literatures report that the statin acts on vascular endothelial cells and vascular endothelial progenitor cells derived from the bone marrow and induces an effect of promoting angiogenesis through these cells.

In a preferable embodiment of the present invention, the drug (peptide hormone) to be encapsulated in nanoparticles is "estrogen". The "estrogen" used in the present invention is a general term of all female hormones having a steroid skeleton and sometimes called as, e.g., a follicular hormone, metabolites thereof, and chemical derivatives thereof (such as homoestrone and dwazinol). Examples of estrogen that can be used in the present invention include 17β-estradiol, estrone, estriol, 17α-ethinylestradiol, equilin, equilenin and a metabolite thereof.

In a preferable embodiment of the present invention, the drug (low-molecular weight compound) to be encapsulated in nanoparticles is "vitamin A or a derivative thereof" and preferably "retinoic acid". The retinoic acid, which is a derivative of vitamin A (retinol), has the same physiological activity as vitamin A; however the level of physiological activity is extremely high compared to vitamin A. It is known that retinoic acid is used for therapeutically treating for, e.g., blemish, acne, wrinkle and keloid (by e.g., whitening and skin regeneration). Note that, the whitening effect by retinoic acid is presumably produced not by suppressing a melanin pigment but by promoting skin turnover.

In a preferable embodiment of the present invention, the drug (low-molecular weight compound) to be encapsulated in nanoparticles is "vitamin C (ascorbic acid) or a derivative thereof". Vitamin C is a vitamin indispensable for synthesis collagen constituting a connective tissue such as skin, tendon and cartilage, and also has, e.g., an effect to inhibit activity of a melanin pigment synthase, i.e., tyrosinase, more specifically an effect to suppress synthesis of a melanin pigment (or thin a melanin pigment generated), an effect to remove active oxygen, an effect to promote blood circulation and an effect to suppress excessive sebum secretion. Because of this, it is known that vitamin C can be used for whitening (e.g., prevention of blemish) as well as for maintaining skin health and therapeutically treating a skin disease such as wound healing. Examples of vitamin C derivatives include a derivative for improving water solubility or lipid solubility of vitamin C. A vitamin C derivative can be selected in consideration of the function effect of the invention; for example, lipid-soluble vitamin C such as ascorbyl palmitate and ascorbyl tetrahexyldecanoate can be used.

The drug to be encapsulated in nanoparticles may be a component used as a whitening agent similarly to vitamin C; for example, a substance having a melanin pigment synthesis suppressive action such as hydroquinone, arbutin, kojic acid, tranexamic acid, resorcinol, linoleic acid, ellagic acid, azelaic acid and fullerene; and an agent having a turnover promoting effect such as a chemical peeling agent (which may contain glycolic acid, salicylic acid, lactic acid and trichloro acetate).

Further, the drug to be encapsulated in nanoparticles may be vitamin B, vitamin E or a vitamin other than those mentioned above. These vitamins produce effects against peripheral nerve injury and peripheral circulatory disturbance, through the skin. Compositions containing vitamin B, vitamin E, placenta and chamomile ET (an extract obtained from chamomile flower by extraction with squalane) are known also as a whitening agent.

In the present invention, drugs may be used singly or in combination of two types or more. More specifically, drug-encapsulating nanoparticles to be incorporated in stem cells for collecting exosomes may encapsulate only a single drug or a combination of drugs (two types or more). Alternatively, the exosomes to be used in the present invention may be exosomes secreted from stem cells containing single drug-encapsulating nanoparticles or may be a mixture of exosomes each secreted from stem cells containing drug-encapsulating nanoparticles containing different drugs. In such embodiment as mentioned, examples of the combination of drugs include a combination of two types or more drugs selected from the group consisting of, e.g., statin, estrogen, vitamin A and a derivative thereof, and vitamin C and a derivative thereof.

In the present invention, the "bioabsorbable polymer" to be used as a material for nanoparticles can be selected from various compounds which can constitute drug-encapsulating nanoparticles. Representative examples of the bioabsorbable polymer include a poly lactic acid (PLA) polymer and a poly(lactic-co-glycolic acid) (PLGA) copolymer. Since PLA is hydrolyzed *in vivo* into lactic acid and PLGA is hydrolyzed *in vivo* into lactic acid and glycol and are both finally into water and carbon dioxide gas, PLA and PLGA are preferable as a harmless material (having no biotoxicity).

In the present invention, drug-encapsulating nanoparticles are produced such that the number average particle size measured by a light scattering method becomes usually less than 1000 nm, preferably 100 nm to 400 nm and more preferably 200 nm to 400 nm, in view of uptake efficiency into stem cells.

The drug-encapsulating nanoparticles may be produced in any method as long as the nanoparticles having the above number average particle size can be obtained; however, spherical crystallization technique is preferably used for production. The spherical crystallization technique is already well known as a method for controlling generation/growth of crystals in a final process of compound synthesis, i.e., a method of designing spherical crystal particles and processing them by directly controlling physical properties thereof. As one of the spherical crystallization techniques, an emulsion solvent diffusion technique (ESD) is well known.

The emulsion solvent diffusion technique is carried out by using two types of organic solvents: a good solvent which can dissolve a bioabsorbable polymer such as PLA or PLGA for use in encapsulating a drug and a poor solvent which does not dissolve the polymer. First, a polymer such as PLA or PLGA is dissolved in a good solvent. Then, a drug solution is added to the good solvent so as for the polymer not to precipitate, and mixed. The solution mixture is added dropwise to a poor solvent being stirred. Since the good solvent and the poor solvent are rapidly diffused to each other, the interface between the organic solvent phase and the aqueous phase is disturbed. As a result, the good solvent is emulsified by itself to form submicron-size emulsion drops. Thereafter, mutual diffusion between the good solvent and the poor solvent further proceeds and the solubilities of a polymer such as PLA or PLGA and the drug within the emulsion droplets decrease, with the result that polymer nanoparticles as spherical crystal particles, including the drug are produced.

### <Stem cells>

In the present invention, the "stem cells" refer to cells having self-replicating ability and totipotency, pluripotency or multipotency and include various types of stem cells known to those skilled in the art. Examples of the stem cells include embryonic stem cells (ES cells) and somatic stem cells such as induced pluripotent stem cells (iPS cell) and mesenchymal stem cells. The stem cells can be distinguished from other cells typically based on whether the expression of a predetermined single or two or more genes or proteins (so-called marker gene(s) or marker protein(s)) is positive or negative.

In the present invention, in order to obtain many types of stem cells simply in a large amount, mesenchymal stem cells obtained from the bone marrow tissue, adipose tissue and the like, particularly "adipose-derived stem cells", i.e., mesenchymal stem cells obtained from the adipose tissue, are preferably used. Single administration of the adipose-derived stem cells is already carried out in clinical sites and known to differentiate into not only adipose but also bone, liver and heart. The adipose-derived stem cells can be easily extracted and separated in a large amount from, e.g., subcutaneous adipose obtained by a minimally invasive technique such as liposuction. For example, the adipose tissue obtained is treated with collagenase, and mononuclear cells alone are collected therefrom by a centrifugal specific gravity method. The collected mononuclear cells are cultured in a culture plate for about 4 days. The resultant adherent cells can be selected as adipose-derived stem cells and isolated. Alternatively, the adipose-derived stem cells can be extracted and separated by use of a full-automatic adipose tissue stem cell extractor such as Celution System (manufactured by Cytori Therapeutics Inc.) from the adipose tissue collected. In view of efficiency (economic point of view), it is extremely advantageous to use adipose-derived stem cells as the stem cells of the present invention.

The stem cells may be cells contained in the tissue collected (primary cultured cells) or cells obtained by subjecting the primary cultured cells serially to passage culture predetermined times (subcultured cells).

The donor for stem cells (or tissue form which stem cells are isolated) may be a human or a non-human mammal (e.g., mouse) and preferably a subject belonging to the same species as those which will use the composition for therapeutically treating a skin disease according to the present invention. Particularly, if the composition for therapeutically treating a skin disease according to the present invention is produced as, e.g., a drug or a cosmetic to be applied to humans (patients), the donor is preferably a human. The donor may be the same subject (autogenous) or a different subject (heterogenous) as the person who uses a composition for therapeutically treating a skin disease. The sex and age of the donor are not particularly limited.

The stem cells containing drug-encapsulating nanoparticles can be prepared simply, for example, by adding drug-encapsulating nanoparticles in a medium having stem cells cultured. The drug-encapsulating nanoparticles in the medium are easily taken up by stem cells through phagocytosis without using, e.g., a special reagent. The concentration of drug-encapsulating nanoparticles in a medium or the weight of drug-encapsulating nanoparticles per predetermined number of stem cells can be appropriately controlled in accordance with, e.g., the type and state (e.g., a number of subcultures) of stem cells to be used and properties of the drug-encapsulating nanoparticles (e.g., type of drug, the weight of drug contained in a predetermined amount of drug-encapsulating nanoparticles) such that a desired therapeutic effect for a skin disease is exerted by the composition containing exosomes secreted.

In an embodiment of the present invention, the concentration of drug-encapsulating nanoparticles in a medium is, for example, 5 to 500 µg/mL, preferably 10 to 200 µg/mL and more preferably 20 to 100 µg/mL. In an embodiment of the present invention, the weight of drug-encapsulating nanoparticles in medium per 50000 stem cells (typically adipose-derived stem cells) is, for example, 5 to 1000 µg, preferably 10 to 500 µg and more preferably 25 to 300 µg. If the encapsulation ratio of a drug in drug-encapsulating nanoparticles (the ratio of a weight of a drug encapsulated in drug-encapsulating nanoparticles to a predetermined weight of the drug-encapsulating nanoparticles) is multiplied by the weight of the above drug-encapsulating nanoparticles, the numeric range of the drug-encapsulating nanoparticles is converted in the numeric range of the drug encapsulated.

The stem cells containing drug-encapsulating nanoparticles are enhanced in functions such as migratory capacity, proliferative capacity and immunosuppressive ability; at the same time, productivity of factors useful for therapeutically treating a skin disease, such as factors involved in angiogenesis (for example, FGF (fibroblast growth factor) such as FGF-2; VEGF (vascular endothelial growth factor) such as VEGF-A and VEGF-C; and HGF (stem cell growth factor)). The exosomes secreted from stem cells enhanced in function as mentioned above and to be used in the present invention contain not only the drug encapsulated in the nanoparticles but also many types of factors produced by the stem cells, in particular angiogenic factors useful for therapeutically treating a skin disease. Note that the nanoparticles constituted of a bioabsorbable polymer, since they are usually decomposed within cells, are not contained in the exosomes secreted outside the cells.

Exosomes can be collected from the medium by a method ordinarily known in the art, after stem cells are cultured, for example, in a medium having drug-encapsulating nanoparticles added therein, for a predetermined period, or, if necessary, from the medium of passage culture repeated predetermined times. The culture period of stem cells in a medium containing drug-encapsulating nanoparticles can be appropriately controlled; however, the culture period is usually about 4 to 5 days. Compared to exosomes collected from the medium containing drug-encapsulating nanoparticles after stem cells are cultured, exosomes collected from the medium (ordinary medium not containing the drug-encapsulating nanoparticles) of serial (one, two or more)-passage culture of stem cells, sometimes contain various factors (e.g., angiogenic factors) produced by the stem cells and/or a drug encapsulated in the nanoparticles in large amounts. If the exosomes to be used have such properties, the exosomes are preferably collected after a requisite number of passage culture. Exosomes can be collected by an ordinary means, for example, a centrifuge.

### <Composition for therapeutically treating skin disease>

In the present invention, the "skin disease" refers to a disease having an abnormal skin condition and a skin damage or refers to a symptom/state of an abnormal skin condition and a skin damage; and includes not only an abnormal skin condition and a skin damage themselves but also an abnormality of hair in connection with the skin. Examples of the skin disease (e.g., symptom and condition) as mentioned include a skin damage caused by, e.g., severe burn, refractory skin ulcer such as pressure ulcer, radiation ulcer, alopecia areata, alopecia such as hair loss as a side effect of an anti-cancer agent and atrichia. Note that the abnormalities and damages (e.g., a symptom and condition) of the skin and hair include those caused by aging.

In the present invention, the "therapeutic treatment" refers to complete or partial cure for a skin disease (disease itself or a symptom and condition associated with a disease); mitigation or improvement of a skin disease; deceleration of progress of a skin disease; delaying or preventing onset of a skin disease; and reducing a risk of onset, singly or in combination. Since such a broad range is conceptually included, the term "therapeutic treatment" should be interpreted as not a synonym of a narrow sense: healing (cure) and can be rephrased by the term "treatment".

Statin itself has an effect to promote angiogenesis as well as acts on stem cells to enhance various functions; particularly can improve an ability to produce angiogenic factors. Accordingly, exosomes secreted from stem cells containing statin-encapsulating nanoparticles are able to contain not only statin itself but also factors and substances capable of promoting angiogenesis in large amounts. If the exosomes as mentioned are contained in an effective amount, composition for therapeutically treating a skin disease, having an excellent effect on alopecia, atrichia and/or other diseases which are effectively therapeutically treated by angiogenesis can be prepared.

Estrogen has an effect to promote production of collagen from fibroblasts and angiogenesis. Accordingly, exosomes secreted from stem cells containing estrogen-encapsulating nanoparticles are able to contain not only estrogen itself but also factors and substances capable of promoting angiogenesis and regeneration of the dermis, in large amounts. If the exosomes as mentioned are contained in an effective amount, a composition for therapeutically treating a skin disease, having an excellent effect on skin damage, skin ulcer and/or other diseases which are effectively therapeutically treated by angiogenesis and regeneration of the dermis can be prepared.

Other than above, if nanoparticles encapsulating vitamin A or a derivative thereof (e.g., retinoic acid) and vitamin C or a derivative thereof (ascorbyl palmitate) are incorporated in stem cells, exosomes to be secreted will contain not only these compounds but also factors having an effect to promote angiogenesis and/or dermal regeneration in large amounts. If such exosomes are used, a composition for therapeutically treating a skin disease, having the excellent effect on diseases in the same as in the case(s) of statin and/or estrogen can be prepared.

In an embodiment of the present invention, a composition for therapeutically treating a skin disease such as alopecia or atrichia contains at least one type of drug selected from the group consisting of statin, vitamin A and a derivative thereof, and vitamin C and a derivative thereof, as the drug to be encapsulated in drug-encapsulating nanoparticles. In an embodiment of the present invention, a composition for therapeutically treating a skin disease such as a skin damage or a skin ulcer contains at least one type of drug selected from the group consisting of estrogen, and vitamin A and a derivative thereof, as the drug to be encapsulated in drug-encapsulating nanoparticles.

The composition for therapeutically treating a skin disease according to the present invention can be produced according to classification as a drug, a cosmetic and a quasi-drug (medicinal cosmetic) positioned between a drug and a cosmetic in accordance with the legal system of each country, or as various compositions directed to various skin diseases as a therapy target. A composition for therapeutically treating a skin disease serving as a (medicinal) cosmetic may be a product directed to anti-aging, for example, for preventing skin aging with aging.

The dosage form or the route of administration for the composition for therapeutically treating a skin disease according to the present invention can be appropriately selected; however, an external preparation that is directly applied to an affected site of the skin; in other words, dosage form for transdermal administration, is suitable. If the composition for therapeutically treating a skin disease is used as an external preparation (for transdermal administration), various components contained in exosomes are allowed to locally act around an affected site. More specifically, since a therapeutic effect can be exerted at relatively low concentrations, side effects which may be produced by systemic administration dosage form containing these components in high concentrations, can be avoided. However, if such a side-effect problem can be avoided, the composition for therapeutically treating a skin disease according to the present invention can be produced as, for example, as injection (for intravenous administration).

Note that a "pharmaceutical composition" for therapeutically treating a skin disease disclosed in the priority document of this application is conceptionally identical with the "composition" for therapeutically treating a skin disease of the present invention that can be produced as a drug, a quasi-drug and a cosmetic.

Examples of dosage form of an external preparation for skin include a liquid, a spray, an ointment, a cream, a gel agent and a patch. Examples of the external preparation for skin serving as a (medicinal) cosmetic include basic cosmetics such as a lotion, a cream, an emulsion, a serum and a facial wash; makeup cosmetics such as a makeup base; hair care products such as a shampoo, a conditioner and hairdressing; and other cosmetics such as a whitening agent and a hair removal agent.

The composition for therapeutically treating a skin disease according to the present invention may comprise exosomes secreted from stem cells containing drug-encapsulating nanoparticles (nanoparticle-containing stem cells) and other components depending on the classification (e.g., a drug, a quasi-drug, a cosmetic) and the dosage form. The content of exosomes in the composition for therapeutically treating a skin disease may be determined as an effective amount for exerting a desired effect as, e.g., a drug, a quasi-drug or a cosmetic, in view of properties of the exosomes to be used (particularly, type and concentration of a component contained in exosomes) and can be appropriately controlled by those skilled in the art based on experiments ordinarily carried out.

As the components other than exosomes, additives ordinarily used in producing drugs, quasi-drugs and cosmetics, such as a stabilizer, a preservative, a buffer, a pH regulator and an excipient (preferably additives for use in producing external preparations (transdermal administration composition)) are mentioned. The contents of components other than exosomes can be appropriately controlled by those skilled in the art.

The composition for therapeutically treating a skin disease according to the present invention can be produced by a production method comprising the steps of: allowing stem cells to contain drug-encapsulating nanoparticles in which a drug is encapsulated in nanoparticles comprising a bioabsorbable polymer; and collecting exosomes secreted from the stem cells. General items regarding to a method for producing a composition for therapeutically treating a skin disease, for example, steps other than the above steps, apparatuses and production conditions may be determined in accordance with the items employed in ordinary methods for producing a composition for therapeutically treating a skin disease or the like, containing exosomes.

### Examples

Now, Examples for describing preparation of drug-encapsulating nanoparticles used in the present invention, stem cells containing the nanoparticles, and production of a composition for therapeutically treating a skin disease comprising exosomes secreted from the nanoparticle-containing stem cells and the therapeutic effect will be described below.

### [Preparation Example 1] Preparation of statin (simvastatin)-encapsulating nanoparticles

Statin-encapsulating nanoparticles using statin (simvastatin) as a drug and a poly lactic acid (PLA) polymer or a poly (lactic-co-glycolic acid)(PLGA) copolymer as a material for the nanoparticles were prepared in accordance with the following procedure.

PLA (weight average molecular weight: 10000) (50 mg) and simvastatin (10 mg) were dissolved in a solution mixture of acetone (2 mL) and ethanol (0.5 mL) to prepare a polymer solution. The polymer solution obtained was added dropwise to 2 wt% PVA solution (25 mL) which was stirred at room temperature at 500 rpm, to obtain a simvastatin-encapsulating PLA nanoparticle suspension. Subsequently organic solvents (acetone and ethanol) were evaporated while the polymer solution was continuously stirred at 500 rpm and room temperature for about 16 hours (16 to 24 hours). After completion of evaporation of the solvents, the suspension was centrifuged at 4°C and 60000 g for 30 minutes. The resultant precipitate was recovered and resuspended in distilled water. The operation of centrifugation/resuspension in distilled water was repeated three times in total. Thereafter, the suspension was lyophilized overnight to obtain a simvastatin-encapsulating PLA nanoparticles. Simvastatin (24.94 µg) was encapsulated in 1 mg of PLA nanoparticles.

Also, simvastatin-encapsulating PLGA nanoparticles were prepared in the same manner as in the above method for preparing simvastatin-encapsulating PLA nanoparticles except that the same amount of PLGA (weight average molecular weight: 6500) was used in place of PLA as the material for nanoparticles.

### [Preparation Example 2] Preparation of estrogen (estradiol: E2)- encapsulating nanoparticles

Estradiol-encapsulating PLGA nanoparticles were prepared in the same manner as in method of preparing simvastatin-encapsulating PLGA nanoparticles according to Preparation Example 1 except that 10 mg of estradiol (E2) was used as a drug in place of 10mg of simvastatin.

### [Preparation Example 3] Preparation of vitamin C-encapsulating nanoparticles

Vitamin C-encapsulating PLGA nanoparticles were prepared in the same manner as in method of preparing simvastatin-encapsulating PLGA nanoparticles according to Preparation Example 1 except that 10 mg of vitamin C (ascorbyl palmitate) was used as a drug in place of 10 mg of simvastatin.

### [Preparation Example 4] Preparation of retinoic acid-encapsulating nanoparticles

Retinoic acid-encapsulating PLGA nanoparticles were prepared in the same manner as in method of preparing simvastatin-encapsulating PLGA nanoparticles according to Preparation Example 1 except that 10 mg of retinoic acid was used as a drug in place of 10 mg of simvastatin.

### [Preparation Example 5] Preparation of rhodamine-encapsulating nanoparticles

Rhodamine-encapsulating PLA nanoparticles and rhodamine-encapsulating PLGA nanoparticles were prepared in the same manner as in methods of preparing simvastatin-encapsulating PLA nanoparticles and simvastatin-encapsulating PLGA nanoparticles according to Preparation Example 1 except that 10 mg of rhodamine (red fluorescent dye) was used as a pseudo drug in place of 10 mg of simvastatin.

### [Reference Example 1] Treatment for adipose-derived stem cells with rhodamine-encapsulating PLA nanoparticles

As a preliminary experiment for Reference Example 2 (Treatment for adipose-derived stem cells with statin (simvastatin)- encapsulating nanoparticles), the optimal concentration of a drug (statin)-encapsulating nanoparticles for treating adipose-derived stem cells was examined herein. For the purpose, the following experiments were carried out using the rhodamine-encapsulating nanoparticles obtained in Preparation Example 5 (containing rhodamine as a placebo).

To carry out the experiments, first, adipose-derived stem cells (AdSC) were obtained each from human adipose tissue and mouse adipose tissue in accordance with an ordinary method using a collagenase treatment and a centrifugal specific gravity method. The procedure will be specifically described below.

### (Isolation of adipose-derived stem cells from human adipose tissue)

A LiberaseTM RG (0.5 mg/mL = 0.47 WU/mL, Sigma5401127001 50 mg/vial)/HBSS (ThermoFisher14175095) solution and 10X hemolysis solution (NH₄Cl 8.3 g, NaHCO₃1.2 g, 0.5 M EDTA solution 200 µL/100 mL) were prepared in advance. Adipose contained in three 50-mL suction syringes (150 mL, collected from a woman of 65 years old) was transferred to a plastic bottle with a nozzle (AS ONE #1-4640-03, wide-mouth washing bottle 1000 ml), washed with the same amount of PBS (-) and discarded. This wash/discard operation was repeated 4 or 5 times. The above Liberase TM RG solution (50 mg/vial was dissolved with 20 mL of HBSS. A half (10 mL) of the resultant solution was diluted with HBSS (40 mL) to obtain a solution of 50 mL in total, which was used for about 150 mL of the adipose tissue) was transferred to the bottle containing the adipose solution. The bottle was lightly vortexed and incubated while shaking in a thermostatic bath equipped with a shaker at 37°C for 20 minutes. Thereafter, 20% FBS/DMEM F12 (50 mL) was added in the bottle to terminate the enzymatic reaction. The liquid layer (lower layer of adipose) was discharged from the nozzle of the bottle, passed through a cell strainer (100 µm, BD) and delivered (collected) to a plurality of unused 50-mL tubes. The tubes were placed in a centrifuge (with a brake) and centrifuged at 1200 rpm (300 g) × 5 min and the supernatant was discarded. The cell pellet was suspended with 1 mM EDTA/PBS (40 mL/tube), passed through a cell strainer (40 µm, BD) and delivered to the same number of unused 50-mL tubes as above. The tubes were placed in a centrifuge (with a brake) and centrifuged at 1200 rpm (300 g) × 5 min and the supernatant was discarded. The cells were suspended with 1 mM EDTA/PBS (2 ml) and collected in a single tube. To the tube, a hemolysis solution (8 mL) was added, and mixed. Thereafter the tube was stored (placed) on ice for 10 minutes (hemolysis operation). After 1 mM EDTA/PBS was added up to about 45 mL, the tube was centrifuged at 1200 rpm (300 g) × 5 min, and the supernatant was discarded. The cell pellet was suspended with PBS (-) or 10% DMEM F12 and cultured in a 5% CO₂ incubator together with 10% DMEMF12 for 3 or 4 days. The resultant adherent cells were used in experiments as adipose-derived stem cells (AdSC) (P0). For passage culture, adipose-derived stem cells were further subjected to passage culture for 3 or 4 days at a density of 1: 3 to 4 (3000-4000/cm²).

### (Isolation of adipose-derived stem cells from mouse adipose tissue)

A collagenase type VIII (2 mg/mL, Sigma #C2139)/1% BSA HBSS solution was thawed in a thermostatic bath equipped with a shaker at 37°C, in advance. Also, 1 mM EDTA/PBS was prepared by diluting EDTA (0.5M EDTA, pH 8.0, LifeTechnologies, AM9260G), 10X DPBS, Ca (-), Mg (-) (GIBCO, 14200-166). Thereafter, 2 or 3 mice were anesthetized, and then, killed by removing blood from the epigastric region by a syringe (e.g., insulin syringe) with a 26 to 29G needle. Subcutaneous adipose (inguinal region to back) was taken and collectively placed in a 3.5 cm-culture dish (a drop of physiological saline was added at the center such that adipose tissue was easily taken out). The adipose tissue, while keeping it on the cover, was cut (about 20 to 30 times) into small pieces by scissors. The adipose tissue pieces were placed in a 15 mL tube together with the same volume of collagenase solution as that of adipose. The tube was covered with a cap, shaken up and down to mix and incubated in a thermostatic bath equipped with a shaker at 37°C for 30 minutes. The enzymatic reaction was terminated by adding a 10% FBS/DMEM F12 medium in the same amount. After the uppermost adipose layer was removed by suction, the supernatant was passed through a cell strainer (40 µm, BD, 352340), collected in an unused 50 mL tube and spun by a centrifuge (with a brake) at 1200 rpm (250 g) × 5 min, and then, the supernatant was discarded. To the tube, 1 mM EDTA/PBS (-) was added up to 10 ml to suspend the cell pellet. The tube was spun by a centrifuge (with a brake) at 1200 rpm (250 g) × 5 min., and then, the supernatant was discarded. The cell pellet was suspended with a 10% FBS/DMEM F12 medium, seeded in a culture dish (P0), cultured in a 5% CO₂ incubator for 3 or 4 days. The adherent cells were regarded as AdSC and subjected to passage culture (P1) at a ratio of 1: 1 in a 5% CO₂ incubator for 4 or 5 days. The adherent cells were regarded as AdSC and subjected to passage culture (P2) at a ratio of 1: 3. At the time when adherent cells whose density increased up to 80 to 90%, they were used in experiments.

### (Uptake of rhodamine-encapsulating nanoparticles)

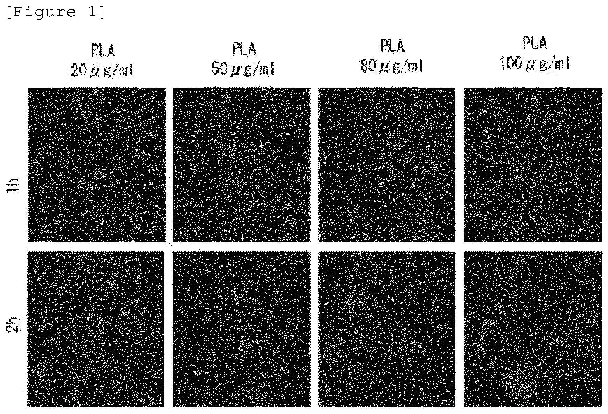

To the medium of human adipose-derived stem cells obtained by the above method, rhodamine-encapsulating PLA nanoparticles obtained in Preparation Example 5 were added so as to obtain a final concentration of 20 µg/mL, 50 µg/mL, 80 µg/mL or 100 µg/mL. One hour (1h) or 2 hours (2h) after addition, uptake of rhodamine-encapsulating PLA nanoparticles was observed by a confocal laser fluorescence microscope. Note that the nuclei were stained with DAPI in accordance with an ordinary method. The results are shown in Figure 1.

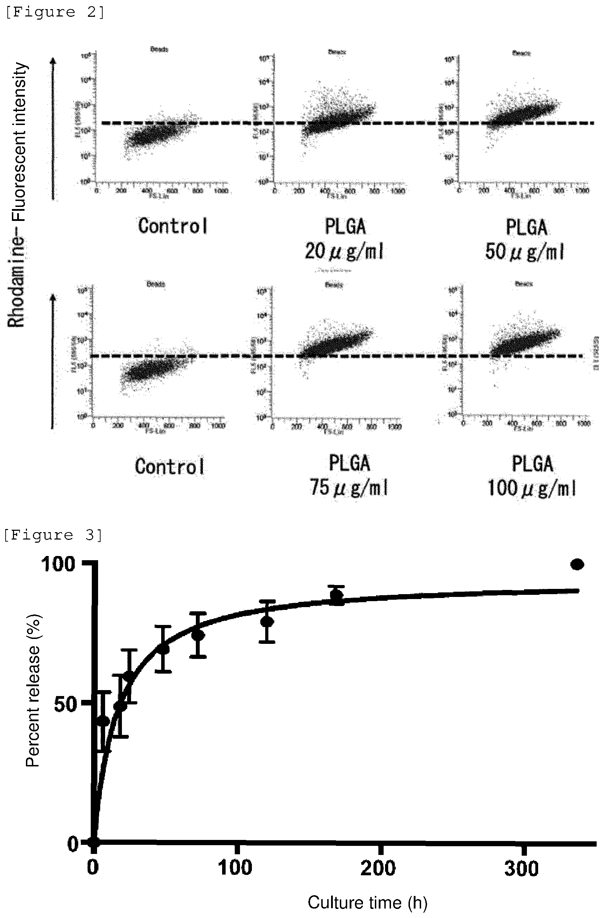

Also, to the medium of mouse adipose-derived stem cells obtained by the above method, rhodamine-encapsulating PLGA nanoparticles obtained in Preparation Example 5 were added so as to obtain a final concentration of 20 µg/mL, 50 µg/mL, 75 µg/mL or 100 µg/mL. Thirty minutes after addition, flow cytometric analysis was carried out by use of a FACS apparatus (BD FACSAria, BD Biosceinces) and the analysis software attached to the apparatus. The results are shown in Figure 2.

As shown in Figure 1, it is found that rhodamine-encapsulating PLA nanoparticles were taken up in human adipose-derived stem cells at any concentration. Note that, the uptake amount of rhodamine-encapsulating PLA nanoparticles by human adipose-derived stem cells increases in a treatment-concentration dependent manner. Particularly at a concentration of 100 µg/ mL, it was confirmed that a large amount of rhodamine-encapsulating PLA nanoparticles are taken up in human adipose-derived stem cells. It is also found that the uptake amount of rhodamine-encapsulating PLA nanoparticles by human adipose-derived stem cells at the two-hour treatment time is larger than one-hour treatment time.

Similarly, as shown in Figure 2, it is found that rhodamine-encapsulating PLGA nanoparticles can be taken up in mouse adipose-derived stem cells at any concentration; and that the uptake amount thereof increases in a treatment-concentration dependent manner.

### [Reference Example 2] Treatment for adipose-derived stem cells with statin (simvastatin)-encapsulating PLA nanoparticles

### (Release of stain from adipose-derived stem cells into the medium)

To examine how to release simvastatin from adipose-derived stem cells containing statin (simvastatin)-encapsulating nanoparticles, the amount of simvastatin released in a medium was measured with the passage of time. The specific procedure is as follows. Simvastatin-encapsulating PLA nanoparticles were added in a culture medium for human adipose-derived stem cells in a concentration of 100 µg/mL and cultured for one hour in the same manner as in Reference Example 1. Thereafter, the medium was exchanged. Six hours, 18 hours, 24 hours, 48 hours, 72 hours, 120 hours, 168 hours and 336 hours after exchange of the medium, the amount of simvastatin, which was released from human adipose-derived stem cells containing simvastatin-encapsulating PLA nanoparticles to the medium, was measured by HPLC (High-pressure Liquid Chromatography). The results are shown in Figure 3.

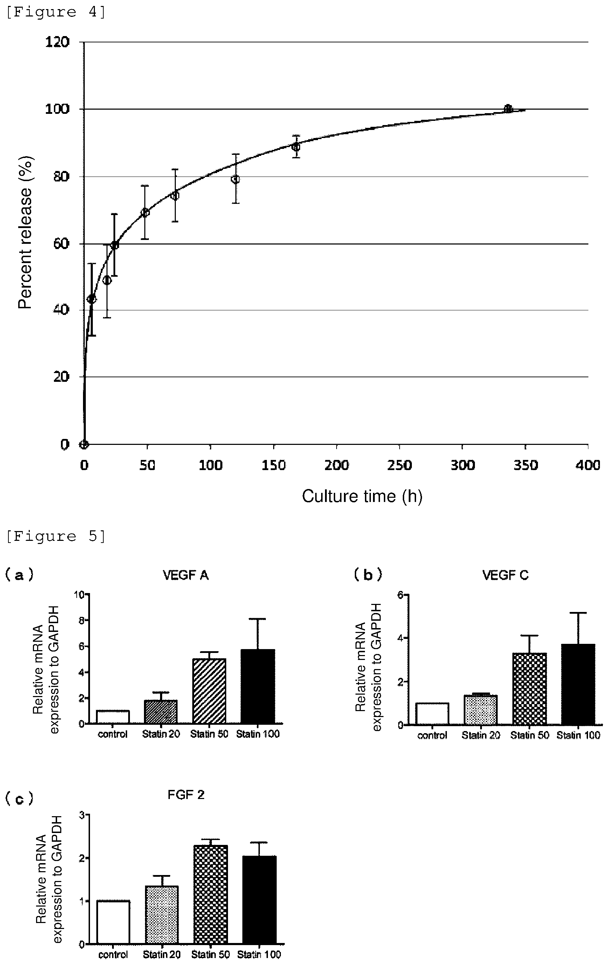

Also, the amount of simvastatin released from mouse adipose-derived stem cells containing simvastatin-encapsulating PLGA nanoparticles to a culture medium was measured in the same manner as above except that simvastatin-encapsulating PLGA nanoparticles were added to the culture medium for mouse adipose-derived stem cells at a concentration of 50 µg/mL and cultured for 30 minutes. The results are shown in Figure 4.

As shown in Figure 3, about 60% of simvastatin was released from human adipose-derived stem cells, during the time from exchange of medium up to 24 hours, and almost all simvastatin was released after about 336 hours. From the result, it is found that simvastatin encapsulated in simvastatin-encapsulating PLGA nanoparticles taken up in human adipose-derived stem cells was gradually and slowly released and almost all simvastatin is released.

In contrast, also in mouse adipose-derived cells, as shown in Figure 4, about 60% of simvastatin was released from mouse adipose-derived stem cells, during the time from exchange of medium up to 24 hours, similarly to the case of human adipose-derived stem cells, and almost all simvastatin was released after about 336 hours. From the result, it is found that simvastatin encapsulated in simvastatin-encapsulating PLA nanoparticles taken up in mouse adipose-derived stem cells was gradually and slowly released and almost all simvastatin is released.

### (Effect on angiogenic factor production ability of adipose-derived stem cells)

To examine the effect of simvastatin-encapsulating nanoparticles on angiogenic factor production ability of adipose-derived stem cells, mRNA expression level of an angiogenic factor in adipose-derived stem cells was analyzed by quantitative PCR method in accordance with the following procedure. In this analysis, angiogenic factors, i.e., VEGF-A, VEGF-C and FGF-2, were subjected to measurement of intracellular mRNA expression level.

First, simvastatin-encapsulating PLA nanoparticles were added in the culture medium for culturing adipose-derived stem cells so as to obtain a concentration of 20 µg/mL, 50 µg/mL or 100 µg/mL. Twenty-four hours later, the cells treated at different concentrations were recovered and total RNA contained in these cells was extracted by NucleoSpinRNA kit (Takara Bio Inc.). From the RNA extracted, cDNA was synthesized by ReverTra Ace qPCR RT kit (TOYOBO). After synthesis, using primers corresponding to the DNA sequences of individual each angiogenic factors (VEGF-A, VEGF-C and FGF-2), mRNA expression levels of VEGF-A, VEGF-C and FGF-2 were measured by quantitative PCR method in each of the cell populations. The quantitative PCR method was carried out by adding SsoFastEvaGreen Mastermix reagent (Bio-Rad) and forward and reverse primers corresponding to each of the angiogenic factors were added in the cDNA solution; and subjecting the resultant mixture to a PCR reaction (95°C 30 seconds: 1 cycle, 95°C 5 seconds/56°C 5 seconds: 40 cycles) by use of a thermal cycler (CFXConnect, Bio-Rad). The measurement results are shown in Figure 5. Note that, the results show the ratios of expression levels of individual factors to the mRNA expression level of GAPDH serving as a standard. The nucleotide sequences of forward and reverse primers used in quantification of VEGF-A, VEGF-C and FGF-2 are as follows:
Forward primer for VEGF-A:
   5'-TTACTCTCACCTGCTTCT-3' (SEQ ID No. 1)
Reverse primer for VEGF-A:
   5'-CTGCTTCTTCCAACAATG-3' (SEQ ID No. 2)
Forward primer for VEGF-C:
   5'-TCAAGGACAGAAGAGACTA-3' (SEQ ID No. 3)
Reverse primer for VEGF-C:
   5'-CCACATCTATACACACCTC-3' (SEQ ID No. 4)
Forward primer for FGF-2:
   5'-TTCTTCCAATGTCTGCTAA-3' (SEQ ID No. 5)
Reverse primer for FGF-2:
   5'-RGACCAATTATCCAAACTGAG-3' (SEQ ID No. 6)

As shown in Figure 5 (a), the mRNA expression level of VEGF-A in the case where simvastatin-encapsulating nanoparticles were treated with any one of concentrations: 20 µg/mL (Statin 20), 50 µg/mL (Statin 50) and 100 µg/mL (Statin 100) was high compared to that of a control group (Control) untreated. In particular, in the case where simvastatin-encapsulating nanoparticles were treated with a concentration of 100 µg/mL, a remarkable increase was observed.

Also, as shown in Figure 5 (b), the mRNA expression level of VEGF-C in the case where simvastatin-encapsulating nanoparticles were treated with any one of concentrations: 20 µg/mL (Statin 20), 50 µg/mL (Statin 50) and 100 µg/mL (Statin 100) was high compared to that of a control group (Control) untreated. In particular, in the case where simvastatin-encapsulating nanoparticles were treated with a concentration of 50 µg/mL or 100 µg/mL, a remarkable increase was observed.

Similarly, as shown in Figure 5 (c), the mRNA expression level of FGF-2 in the case where simvastatin-encapsulating nanoparticles were treated with any one of concentrations: 20 µg/mL (Statin 20), 50 µg/mL (Statin 50) and 100 µg/mL (Statin 100) was high compared to that of a control group (Control) untreated. In particular, in the case where simvastatin-encapsulating nanoparticles were treated with a concentration of 50 µg/mL or 100 µg/mL, a remarkable increase was observed. From these results, it was demonstrated that angiogenic factor production ability of adipose-derived stem cells are enhanced by simvastatin-encapsulating PLA nanoparticles.

As described above, it was confirmed that angiogenic factor production ability of adipose-derived stem cells are enhanced by treatment with statin-encapsulating nanoparticles, and that statin (encapsulated in statin-encapsulating nanoparticles) taken up in adipose-derived stem cells is released from the cells with the passage of time. The results of Reference Example 2 demonstrates that adipose-derived stem cells containing statin-encapsulating nanoparticles can be used for producing a composition for therapeutically treating a skin disease, comprising a drug encapsulated in nanoparticles as well as many components effective for therapeutically treating a skin disease.

### [Example 1] A composition for therapeutically treating a skin disease (e.g., alopecia), comprising exosomes secreted from adipose-derived stem cells containing statin (simvastatin)-encapsulating nanoparticles

A composition for therapeutically treating a skin disease, comprising exosomes secreted from adipose-derived stem cells containing statin-encapsulating nanoparticles was produced in accordance with the following procedure and the therapeutic effect of the composition on the skin disease (e.g., alopecia) was evaluated.

### (Production of test composition S1)

Human adipose-derived stem cells (1 × 10⁶ cells) (see, Reference Example 1) were cocultured with 2 mg of statin-encapsulating nanoparticles in which simvastatin is encapsulated in PLGA nanoparticles (simvastatin-encapsulating PLGA nanoparticles; see Preparation Example 1) for one hour. Thereafter, the human adipose-derived cells were cultured for 72 hours and the culture medium was collected and subjected to ultracentrifugation (210,000 RCF) to separate exosomes from the medium. The exosomes separated were added in a 5% aqueous glycerin solution and mixed to prepare a test composition S1.

### (Evaluation of therapeutic effect of test composition S1 on, e.g., alopecia)

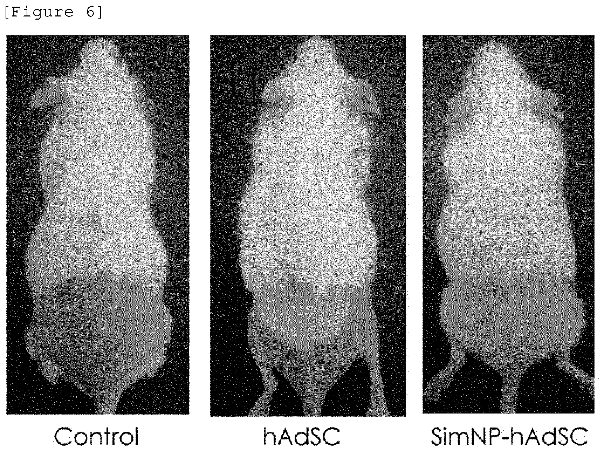

ICR mice were anesthetized by intraperitoneal administration of 300 mg/kg tribromoethanol and fixed in a supine position. On the skin of the back of one of the mice, test composition S1 was applied once. One week later, the condition of the skin was observed. Note that, to one of the ICR mice, a 5% aqueous glycerin solution (control composition A1) not comprising exosomes was applied. To the other ICR mouse, a 5% aqueous glycerin solution (control composition A2) comprising exosomes from human adipose-derived cells not treated with statin-encapsulating nanoparticles, was applied. These were used as controls for use in comparison for the condition of the skin. The results are shown in Figure 6.

As shown in Figure 6, in the ICR mouse (Control) applied with control composition A1, no hair growth was observed. In the ICR mouse (hAdSC) applied with control composition A2, hair growth was slightly observed. In contrast, in the ICR mouse (SimNP-hAdSC) applied with test composition S1, hair growth was observed over the entire back.

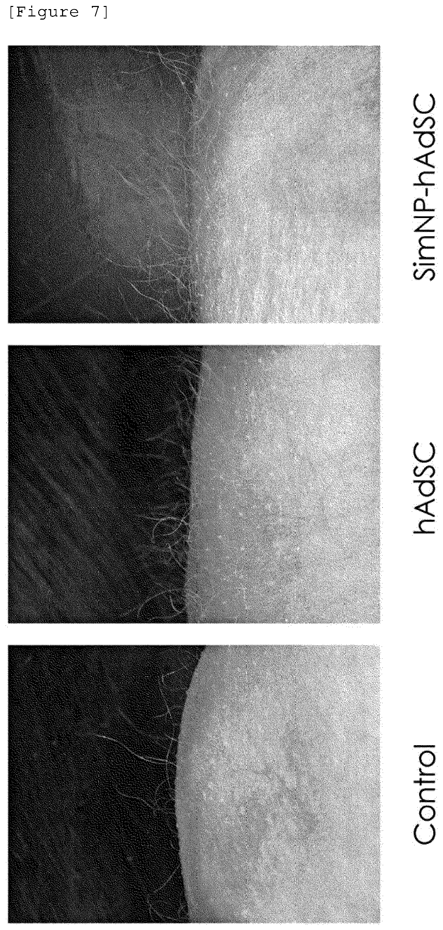

To further evaluate hair growth and hair restoration effects of individual compositions, the same test as above was carried out using hairless mice (HR-1) having a mutation in intron 6 of Hr gene of the 14th chromosome. More specifically, the mice were anesthetized in the same manner as in the aforementioned experiment, and fixed in a supine position. To the skins on the back side of HR-1 mice, the same test composition S1 as above, control compositions A1 and A2 were applied once, respectively. Two weeks later, the conditions of the skins were observed and compared. The results are shown in Figure 7.

As shown in Figure 7, in the HR-1 mouse (Control) applied with control composition A1, hair growth was not observed. Also, in the HR-1 mouse (hAdSC) applied with control composition A2, growth of body hair was slightly observed compared to Control. In contrast, in HR-1 mouse (SimNP-hAdSC) applied with test composition S1, further growth of body hair was observed compared to hAdSC; in other words, a hair growth promoting effect was observed.

From the results, since exosomes from adipose-derived cells treated with statin-encapsulating nanoparticles have high hair-growth and hair-restoration effects, it is concluded that the exosomes are effective for, e.g., alopecia and atrichia.

### [Example 2] Composition for therapeutically treating a skin-disease (e.g., skin damage), comprising exosomes secreted from adipose-derived stem cells containing statin (simvastatin)-encapsulating nanoparticles or estrogen (estradiol: E2)-encapsulating nanoparticles

### (Production of test composition E1)

Exosomes of human adipose-derived stem cells were obtained in the same manner as in Example 1 except that estradiol-encapsulating PLGA nanoparticles (see, Preparation Example 2) were used in place of simvastatin-encapsulating PLGA nanoparticles (see, Preparation Example 1). The exosomes were added in a 5% aqueous glycerin solution and mixed to prepare test composition E1.

### (Evaluation of therapeutic effect of test composition S1 and E1 on e.g., skin damage)

DDY mice were previously anesthetized and fixed in a prone position. After the left and right abdominal walls on the back side thereof were dissected, both ovaries were excised out. The mice were normally reared for 2 months to prepare ovariectomy DDY mice. In the ovariectomy DDY mice, the skin tissue regenerative potential is characteristically low.

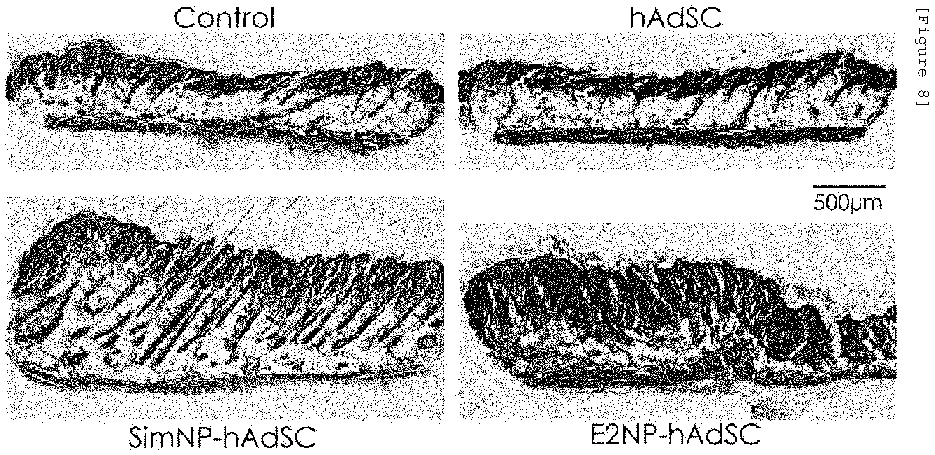

To the skins of ovariectomy DDY mice, the same test composition S1 as used in Example 1 and a test composition E1 newly prepared in the same manner as above in Example 2 were applied respectively at a frequency of 3 times/week. Two weeks after initiation of application, the skin of each of the mice was partially taken, fixed with a 4% paraformaldehyde solution for 6 hours, and sectioned in accordance with an ordinary method to prepare sliced thin-tissue specimens, which were observed by an optical microscope. The skin tissue of the sliced thin-tissue specimens were stained with Masson's trichrome stain to stain fibroblasts producing collagen. Note that, similarly to Example 1, ovariectomy DDY mouse was applied with a 5% aqueous glycerin solution not comprising exosomes (control composition A1) and ovariectomy DDY mouse was applied with a 5% aqueous glycerin solution (control composition A2) comprising exosomes of human adipose-derived cells treated neither with statin-encapsulating nanoparticles nor estradiol-encapsulating PLGA nanoparticles as control mice. The skin conditions were compared with the controls. The results are shown in Figure 8.

As shown in Figure 8, in the ovariectomy DDY mouse (Control) applied with control composition A1, the entire skin was thin, particularly, the fibroblast layer (black portion in the figure) on the side of the epidermis (upper side of the figure) was also thin. In the ovariectomy DDY mouse (hAdSC) applied with control composition A2, difference with Control was not virtually observed. In contrast, in ovariectomy DDY mouse (SimNP-hAdSC) applied with test composition S1, the skin tissue was thick compared to that of Control and, in addition, hair growth was observed. Also, in ovariectomy DDY mouse (E2NP-hAdSC) applied with test composition E1, the skin tissue was thick compared to that of Control and, in particular, a large number of fibroblasts producing collagen (black portion in the figure) were observed.

From the results, it is found that exosomes secreted from stem cells (adipose-derived stem cells) containing drug-encapsulating nanoparticles have a skin regeneration effect. In particular, if exosomes obtained by using estradiol (E2) as a drug, are used, it is found that fibroblasts producing collagen important for skin regeneration can be increased. Because of this, it is concluded that composition comprising exosomes according to the present invention is useful for therapeutically treating skin damage or skin ulcer.

### [Example 3] Composition for therapeutically treating a skin disease, comprising exosomes secreted from adipose-derived stem cells containing estrogen (estradiol: E2)-encapsulating nanoparticles

Human adipose-derived stem cells (AdSC) (primary cultured cells) were isolated from the adipose tissue taken from a women of 71 years old, in the same procedure in "Isolation of adipose-derived stem cells from human adipose tissue" in Reference Example 1. The primary cultured cells were serially subcultured 9 times to prepare a cell population (referred to as "P0" in this Example).

A medium was prepared by adding estradiol-encapsulating PLGA nanoparticles (see, Preparation Example 2) to a mixed medium containing DMEM F12 (manufactured by Thermo Fisher) and KBM ADSC2 (manufactured by KOHJIN BIO Corporation) in a ratio of 1: 1 so as to obtain a concentration of 25, 50, 100, 200 or 300 µg/50000AdSC. Stem cell population P0 was cultured by using the medium thus prepared in a CO₂ incubator of 37°C for 5 days. (stem cell population obtained from the passage culture is referred to as "P1"). After completion of the passage culture, the medium was collected and subjected to ultracentrifugation (210,000 RCF). In this manner, exosomes secreted from stem cell population P1 were separated from the medium, added in a 5% aqueous glycerin solution and mixed to prepare a test composition.

A stem cell population P1 was cultured by using the medium prepared above for 5 days in the same culture conditions as above (stem cell population obtained in the passage culture is referred to as "P2"). Exosomes secreted from stem cell population P2 were separated from the medium in the same procedure as above to prepare a test composition. Thereafter, stem cell populations P3, P4 and P5 were serially prepared by passage culture of 5 days per time. Exosomes secreted were separated from the mediums of P3, P4 and P5 to prepare test compositions.

The stem cell populations (P1, P2, P3, P4 and P5), which secreted exosomes used in producing the individual test compositions, were used as samples and the intracellular mRNA expression levels of VEGF-A, HGF and FGF-2 were measured in the same procedure (quantitative PCR method) as in measurement of intracellular mRNA expression level in Reference Example 2. The forward and reverse primers used in quantification for VEGF-A and FGF-2 are the same as in Reference Example 2. The nucleotide sequences of forward and reverse primers used in quantification for HGF are as follows:
Forward primer for HGF:
   5'-CCTCTATGAAAACAAAGACTAC-3' (SEQ ID No. 7)
Reverse primer for HGF:
   5'-CTGTGTTCGTGTGGTATC-3' (SEQ ID No. 8)

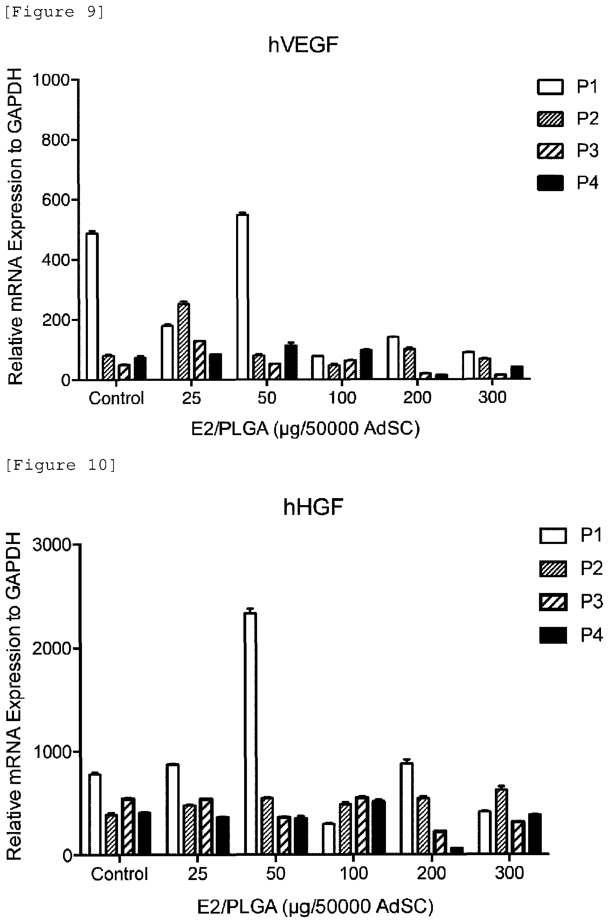

The measurement results of VEGF-A mRNA expression level in the adipose-derived stem cells containing estrogen-encapsulating nanoparticles are shown in Figure 9. From the results, it is estimated that exosomes secreted from the stem cell population P1, which was cultured in a medium having estradiol-encapsulating PLGA nanoparticles added therein so as to obtain a concentration of 50 µg/50000AdSC, contain large amounts of VEGF-A protein translated from mRNA and estradiol encapsulated in the nanoparticles; and that the test composition prepared by using the exosomes has an excellent efficacy in therapeutic treatment (particularly skin regeneration) for a skin disease.

The measurement results of HGF mRNA expression level in the adipose-derived stem cells containing estrogen-encapsulating nanoparticles are shown in Figure 10. From the results, it is estimated that exosomes secreted from the stem cell population P1, which was cultured in a medium having estradiol-encapsulating PLGA nanoparticles added therein so as to obtain a concentration of 50 µg/50000AdSC, contain large amounts of HGF protein translated from mRNA and estradiol encapsulated in the nanoparticles; and that the test composition prepared by using the exosomes has an excellent efficacy in therapeutic treatment (particularly skin regeneration) for a skin disease.

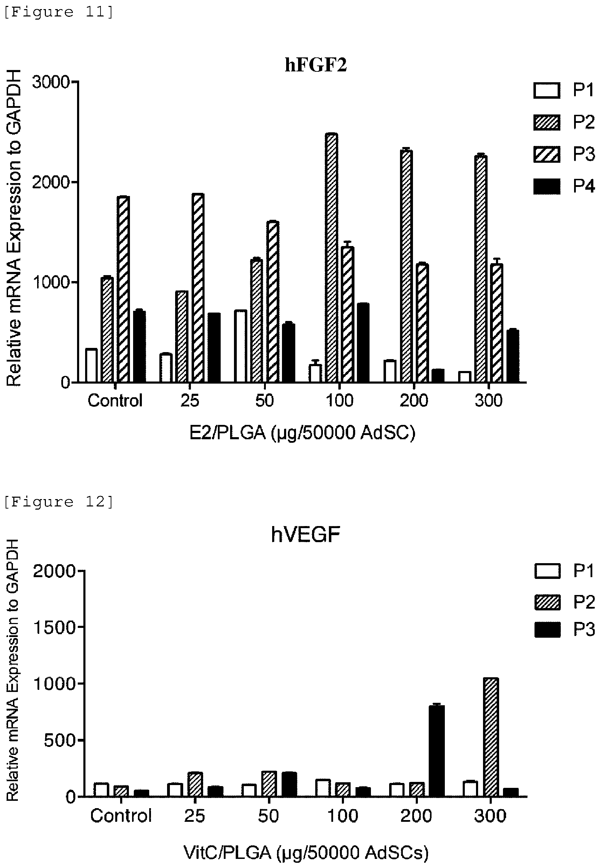

The measurement results of FGF-2 mRNA expression level in the adipose-derived stem cells containing estrogen-encapsulating nanoparticles are shown in Figure 11. From the results, it is estimated that, for example, exosomes secreted from the stem cell population P2, which was cultured in a medium having estradiol-encapsulating PLGA nanoparticles added therein so as to obtain a concentration of 300 µg/50000AdSC, contain large amounts of FGF-2 protein translated from mRNA and estradiol encapsulated in the nanoparticles; and that the test composition prepared by using the exosomes has an excellent efficacy in therapeutic treatment (particularly skin regeneration) for a skin disease.

### [Example 4] Composition for therapeutically treating a skin disease, containing exosomes secreted from adipose-derived stem cells containing vitamin C-encapsulating nanoparticles

Stem cell populations P1 to P5 were prepared in the same manner as in Preparation Example 3 by passage culture (culture period: 5 days per time) except that vitamin C-encapsulating PLGA nanoparticles (see, Preparation Example 3) were used in place of the estradiol-encapsulating PLGA nanoparticles (see Preparation Example 2). Exposomes secreted were separated from each of the mediums to prepare test compositions. Furthermore, the VEGF-A, HGF and FGF-2 intracellular mRNA expression levels of each of stem cell populations P1 to P5 of Example 4 were measured in the same manner as in Example 3.

The measurement results of VEGF-A mRNA expression level in the adipose-derived stem cells containing vitamin C-encapsulating nanoparticles are shown in Figure 12. From the results, it is estimated that exosomes secreted from the stem cell population P2, which was cultured in a medium having estradiol-encapsulating PLGA nanoparticles added therein so as to obtain a concentration of 300 µg/50000AdSC contain large amounts of VEGF-A protein translated from mRNA and vitamin C contained in the nanoparticles; and that the test composition prepared by using the exosomes has an excellent efficacy in therapeutic treatment for a skin disease.

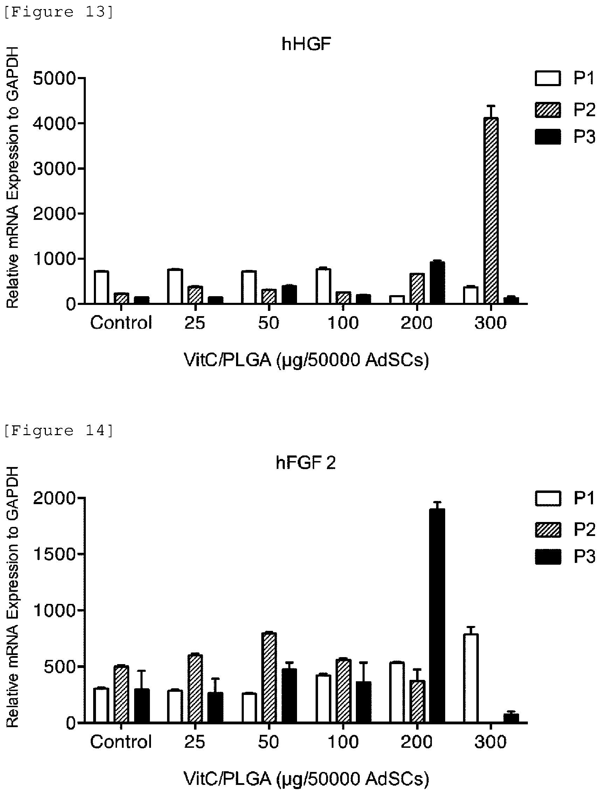

The measurement results of HGF mRNA expression level in the adipose-derived stem cells containing vitamin C-encapsulating nanoparticles are shown in Figure 13. From the results, it is estimated that exosomes secreted from the stem cell population P2, which was cultured in a medium having estradiol-encapsulating PLGA nanoparticles added therein so as to obtain a concentration of 50 µg/50000AdSC, contain large amounts of HGF protein translated from mRNA and vitamin C contained in the nanoparticles; and that the test composition prepared by using the exosomes has an excellent efficacy in therapeutic treatment (particularly skin regeneration) for a skin disease.

The measurement results of FGF-2 mRNA expression level in the adipose-derived stem cells containing vitamin C-encapsulating nanoparticles are shown in Figure 14. From the results, it is estimated that exosomes secreted from the stem cell population P3, which was cultured in a medium having estradiol-encapsulating PLGA nanoparticles added therein so as to obtain a concentration of 200 µg/50000AdSC, contain large amounts of FGF-2 protein translated from mRNA and vitamin C contained in the nanoparticles; and that the test composition prepared by using the exosomes has an excellent efficacy in therapeutic treatment (particularly skin regeneration) for a skin disease.

### [Example 5] Composition for therapeutically treating a skin disease, comprising exosomes secreted from adipose-derived stem cells containing retinoic acid-encapsulating nanoparticles

Stem cell populations P1 to P5 were prepared in the same manner as in Example 3 by passage culture (culture period: 5 days per time) except that retinoic acid-encapsulating PLGA nanoparticles (see, Preparation Example 4) were used in place of the estradiol-encapsulating PLGA nanoparticles (see Preparation Example 2) and the concentrations of the drug-encapsulating nanoparticles in medium were changed from 5 stages (25, 50, 100, 200 and 300 µg/50000AdSC) to 3 stages (25, 50 and 100 µg/50000AdSC). Exposomes secreted were separated from each of the mediums to prepare test compositions. Furthermore, the VEGF-A, HGF and FGF-2 intracellular mRNA expression levels of stem cell populations P1 to P5 of Example 5 were measured in the same manner as in Example 3.

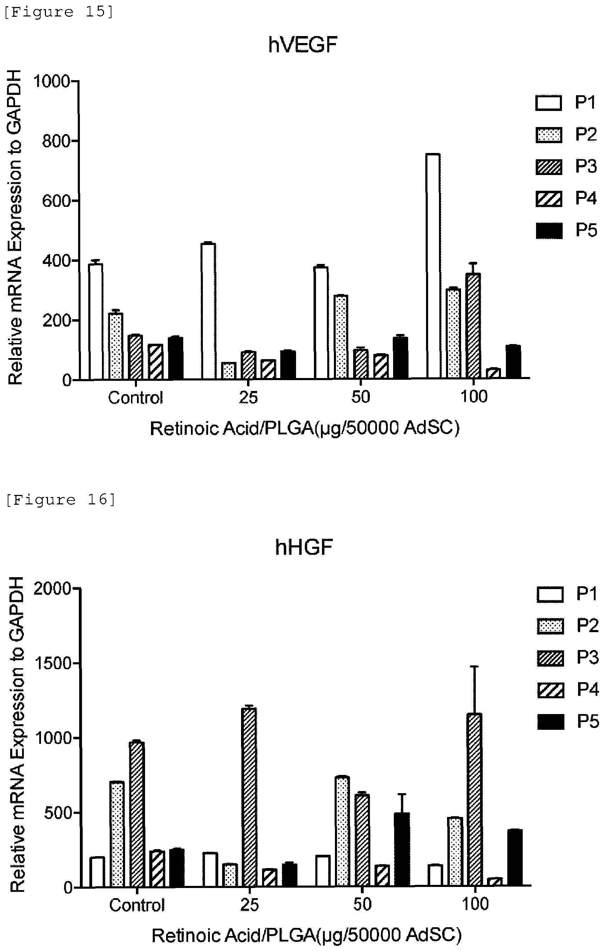

The measurement results of VEGF-A mRNA expression level in the adipose-derived stem cells containing retinoic acid-encapsulating nanoparticles are shown in Figure 15. From the results, it is estimated that, for example, exosomes secreted from the stem cell population P1, which was cultured in a medium having estradiol-encapsulating PLGA nanoparticles added therein so as to obtain a concentration of 100 µg/50000AdSC, contain large amounts of VEGF-A protein translated from mRNA and retinoic acid encapsulated in the nanoparticles; and that the test composition prepared by using the exosomes has an excellent efficacy in therapeutic treatment for a skin disease.

The measurement results of HGF mRNA expression level in the adipose-derived stem cells containing retinoic acid-encapsulating nanoparticles are shown in Figure 16. From the results, it is estimated that exosomes secreted from the stem cell population P3, which was cultured in a medium having estradiol-encapsulating PLGA nanoparticles added therein so as to obtain a concentration of 100 µg/50000AdSC, contain large amounts of HGF protein translated from mRNA and retinoic acid encapsulated in the nanoparticles; and that the test composition prepared by using the exosomes has an excellent efficacy in therapeutic treatment (particularly skin regeneration) for a skin disease.

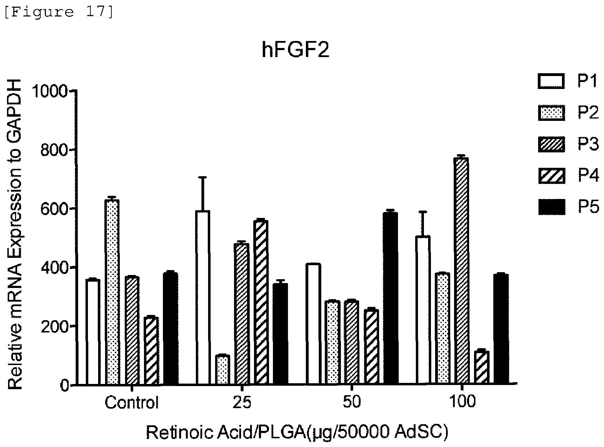

The measurement results of FGF-2 mRNA expression level in the adipose-derived stem cells containing retinoic acid-encapsulating nanoparticles are shown in Figure 17. From the results, it is estimated that exosomes secreted from the stem cell population P3, which was cultured in a medium having estradiol-encapsulating PLGA nanoparticles added therein so as to obtain a concentration of 100 µg/50000AdSC, contain large amounts of FGF-2 protein translated from mRNA and retinoic acid encapsulated in the nanoparticles; and that the test composition prepared by using the exosomes has an excellent efficacy in therapeutic treatment (particularly skin regeneration) for a skin disease.

## Claims

1. A composition for therapeutically treating a skin disease, comprising exosomes secreted from stem cells, wherein
the stem cells are stem cells containing drug-encapsulating nanoparticles in which a drug is encapsulated in nanoparticles comprising a bioabsorbable polymer.

2. The composition according to Claim 1, wherein the stem cells are adipose-derived stem cells.

3. The composition according to Claim 1 or 2, wherein the drug is effective for therapeutically treating a skin disease and enhances an expression level of a factor, which is produced by the stem cells and effective for therapeutically treating the skin disease.

4. The composition according to any one of Claims 1 to 3, wherein the drug is statin.

5. The composition according to any one of Claims 1 to 3, wherein the drug is estrogen.

6. The composition according to any one of Claims 1 to 3, wherein the drug is vitamin A or a derivative thereof.

7. The composition according to any one of Claims 1 to 3, wherein the drug is vitamin C or a derivative thereof.

8. The composition according to any one of Claims 1 to 4, 6 and 7, wherein the skin disease is alopecia or atrichia.

9. The composition according to any one of Claims 1 to 3, 5 and 6, wherein the skin disease is skin damage or skin ulcer.

10. The composition according to any one of Claims 1 to 9, having dosage form for transdermal administration.

11. The composition according to any one of Claims 1 to 10, wherein the composition is a drug, a quasi-drug or a cosmetic.
